(19) Europäisches Patentamt
European Patent Office
Office européen des brevets

(11) Publication number: **0 067 597**
**A1**

(12) # EUROPEAN PATENT APPLICATION

(21) Application number: 82302775.0

(22) Date of filing: 28.05.82

(51) Int. Cl.$^3$: **C 07 H 21/00**

(30) Priority: 29.05.81 US 268238
29.05.81 US 268239

(43) Date of publication of application:
22.12.82 Bulletin 82/51

(84) Designated Contracting States:
AT BE CH DE FR GB IT LI LU NL SE

(71) Applicant: ens BIO LOGICALS INC.
7 Hinton Avenue North
Ottawa Ontario K1Y 4P1(CA)

(72) Inventor: Bender, Robert
7 Hinton Avenue North
Ottawa, Ontario K1Y 4P1(CA)

(72) Inventor: Ogilvie, Kevin K.
54 Place de Bretagne
Quebec J5R 3M8(CA)

(74) Representative: Eyles, Christopher Thomas et al,
BATCHELLOR, KIRK & EYLES 2 Pear Tree Court
Farringdon Road
London, EC1R 0DS(GB)

(54) **Polynucleotide synthesis procedure.**

(57) There is provided a process of preparing oligodeoxyribonucleotides which comprises incorporating ribonucleotide units at specific locations in deoxyribonucleotide chains, to provide predetermined cleavage sites which allow ease of chain cleavage. In one embodiment, a ribo unit is used as the α-unit to couple directly to a polymer support in synthesis, to increase the polymer-nucleoside initial loading. In another embodiment ribo units are used near the ω-end of the chain, followed by a small terminal nucleotide sequence which can be used to separate the product from unwanted products by means of an affinity column. The polynucleotide chain can be treated with base or RNAse, to remove the ribo units therefrom after synthesis and purification.

EP 0 067 597 A1

POLYNUCLEOTIDE SYNTHESIS PROCEDURE

This invention relates to oligodeoxyribonucleotides, and processes for their preparation, and to intermediate reagents for use in synthesis of oligodeoxyribonucleotides.

Oligodeoxyribonucleotides, i.e. compounds consisting of from about 4 to about 80 deoxyribonucleotide units linked together to form a nucleotide chain, are effectively portions of deoxyribonucleic acid (DNA) and thus constitute biologically important products, e.g. as synthetic genes. Synthesis of oligodeoxyribonucleotides with exact, predetermined sequences of units in the chain provides materials capable of being spliced into natural DNA in living organisms, e.g. to replace defective natural genetic material or to modify a living cell so as to provide it with the capability to produce useful secretory products on an enhanced scale. For example, insulin-producing genes, synthetic oligodeoxyribonucleotides of correct unit sequence, have been synthesized and introduced into appropriate cells, to enhance insulin production thereby.

It is however necessary to provide reliable, economical processes for producing oligodeoxyribonucleotides, if such production procedures are to assume greater commercial significance. Synthetic oligodeoxyribonucleotides must be carefully prepared, in step-wise, controlled fashion, to ensure the production of a material with the exactly predetermined unit sequence in its chain. The presence of erroneous chain sequences, omission of a unit from its proper position along the DNA chain and similar, apparently minor defects, can have potentially disastrous consequences, since the unit sequences in the chain carry at least some of the information determining the nature of the secretory products and other behaviour of the cell into which it has been introduced.

One promising method for producing oligonucleotides of predetermined sequence is the polymer support synthesis, described in United States patent application serial No. 06/149,865, of Ogilvie and Bender, filed May 14, 1980. In this method, a solid polymer such as silica gel is derivatized to put functional side groups thereon, and the polymer is reacted with a first, appropriately protected nucleoside unit, to bond that unit to the solid polymer "support". Then, after suitable deprotection of the linked unit, it is phosphorylated and chain extended sequentially with phosphorylated nucleotide compounds. By repeating the sequence of operations, a nucleotide chain can be built up of substantially any desired length, e.g. 150 units long, and with predetermined unit sequences. As a final or close to final step, the oligonucleotide chain is cleaved from the polymer and isolated.

A particular advantage of the above-described process is that it provides the possibility of semi-automatic operation. The polymer-supported product, which is solid, can be kept in a single reaction vessel such as a column throughout the operations, and the various reagents, solvents and wash liquids added thereto from storage vessels in programmed sequence and for pre-set durations. The nucleotide reagents for addition to form units of the DNA-type chain are only a total of four in number. They can be prepared ahead of time, ready for addition to the polymer-supported intermediate product, stored in reagent vessels, and fed to the reaction column as and when required.

In the present invention, polydeoxyribonucleotides are synthesised, using a polymer support process with sequential chain extensions. To enhance the process, however, one or more ribonucleotide units are inserted at specific

locations in the nucleotide chain, to produce intermediate products having, in the chain sequence, both deoxyribo units and one or more ribo units. The use of ribo units in stepwise polymer support synthesis of polydeoxyribonucleotides has been found to give several significant advantages. A ribonucleotide is much less chemically stable than a deoxyribonucleotide, although basically very similar in structure and nature of chemical reactions. Thus it is a relatively straightforward procedure to incorporate a ribonucleotide unit into a deoxyribonucleotide chain when conducting a step-wise chain extension synthesis. The incorporated ribonucleotide unit then acts as a label or marker in the chain, and provides a point of weakness at which the chain can be preferentially cleaved by chemical means. Ribo units are readily subsequently detached from deoxyribo units by enzymatic cleavage using ribonuclease. These factors can be put to practical use in several ways, e.g. enhancement of yield of final product using the polymer support method, ease of separation of final product from by-product, and removal of the products from the polymer support.

Thus according to the present invention, there is provided an oligonucleotide of predetermined unit sequence and chain length and comprising predominantly 3'-5' linked deoxyribonucleotide sequences but having at least one ribonucleotide unit chemically linked thereto at or adjacent to one end of the nucleotide chain, said oligonucleotide corresponding to the general formula:

$$(DNu)_n - RNu - (Nu)_m$$

wherein: $(DNu)_n$ represents a 3'-5' linked deoxyribonucleotide sequence of predetermined unit sequence and length, optionally chemically protected, n deoxyribonucleotide units in length;

n is an integer from 3 to about 200;

RNu represents a ribonucleotide unit;

Nu represents a sequence of either deoxyribonucleotide units or ribonucleotide units, of predetermined unit sequence and chain length, optionally chemically protected, m units in length;

and m is an integer from 0 to 8.

One embodiment of the present invention provides a stepwise nucleotide synthesis process of the polymer support type previously described, in which a single ribo unit followed by a predetermined sequence of at least two ribo units or at least three deoxyribo units is introduced as the final units in the chain, i.e. at the $\omega$-position, remote from the polymer support. The remainder of the chain is predominantly deoxyribonucleotide units, of predetermined sequence and chain length. This enables ready separation of desired product from unwanted but similar by-products.

Such separation of desirable product from undesirable product has previously entailed the use of additional, time consuming reaction steps. For example, if all of the growing DNA chain molecules in the reaction mixture at any particular stage do not undergo the prescribed reaction at the prescribed time, unwanted product will result. A chain may not extend upon addition of the next predetermined nucleotide unit. If this occurs, normally the chain will become inactive and not extend further even during subsequent reaction stages. Nevertheless, it constitutes unwanted byproduct and must be separated from the desirable product at some stage. Since it will have a chemical composition very similar to that of the desirable product, and may even have a similar chain length and molecular weight, its separation has previously proved difficult and time-consuming, especially for long chains, e.g. greater than 20 units length.

Thus there is formed, in one aspect of the present invention, an intermediate product of general formula:

$$Pol - X - (DNu)_m - RNu - (pNu) \qquad (I)$$

in which Pol represents a solid polymer support, suitably derivatized to link to a ~~ribo~~nucleotide unit;

X represents a polymer-linking group;

(DNu) represents a 3', 5'-linked deoxyribonucleotide chain of predetermined unit sequence and chain length;

RNu represents a ribonucleoside or ribonucleotide unit;

(pNu) represents either a predetermined sequence of at least two 3'-5' linked ribonucleotide or ribonucleoside units, or a predetermined sequence of at least three 3'-5' linked deoxyribonucleotide or deoxyribonucleoside units and

m is an integer of at least 3;

This product can be treated with various chemical reagents such as mild alkali (primary amines e.g.tert. butylamine, fluoride ion e.g. tert. butylammonium fluoride) to cleave the solid polymer from the nucleotide chain and thus leave another intermediate product of general formula:

$$Y - (DNu)_m - RNu - (pNu) \qquad (II)$$

where Y is the residue of the linking group X.

Preferably (pNu) represents ribo units so that the chain terminates in a ribose sequence of at least 3 units.

The process and products of this aspect of the present invention afford a number of significant practical advantages in deoxyribopolynucleotide synthesis. Perhaps most importantly, they facilitate the separation of the end product from byproducts, in a simple but extremely efficient manner, and ensure that only the desired end product is recovered. They enable the use for this purpose of standard,

commercially available separating media, namely affinity columns containing oligoribonucleotides attached to solid supports (normally cellulose). The desired products are "tagged" by means of the -sequence of at least three ribo units, and the means by which they are tagged is the same means by which they are separated. The ribo units are chosen in conjunction with those on the affinity column, for the selective separation of the tagged, desired product only. A terminal sequence of deoxyribo units can also clearly be used, along with a deoxyribo affinity column, provided that a ribo unit is included as a separation or cleavage group.The nature of the tags used, ribonucleotide units, in the preferred embodiment enables their ready removal, enzymatically or with alkali, to yield the desired product.

Deoxyribo units will find affinity with an affinity column of the commercially available type, having suitably chosen oligoribonucleotide units thereon, but can only be used conveniently as the w-terminal sequence herein when a ribose unit is incorporated for cleavage purposes. This is less convenient in practice.

In another preferred embodiment, the present invention provides a stepwise nucleotide synthesis process of the polymer support type previously described, but in which a ribonucleotide unit is introduced at the $\alpha$ position, i.e. linked directly to the solid polymer support, through its 5'-position. The succeeding units of chain comprise deoxyribonucleotide units, of predetermined sequence and chain length. It has been found that high yields of initial polymer-nucleoside linked product can be formed, when the nucleoside is a ribose unit bound via its 5'-position. The polymer loadings are more than twice those obtained when a deoxyribose unit is initially bound to the polymer, with consequent

increases in potential final yield of oligonucleotide. This is an important feature, since the initially bound nucleoside units each initiate a chain of oligonucleotide as the synthesis proceeds. To obtain high final yields of oligonucleotide, therefore, one must first obtain high polymer loading with first unit.

Thus there is formed, in the present invention, an intermediate product of general formula:

$$\text{Pol} - (\text{RNu})-(\text{DNu})_m \qquad (I)$$

in which Pol represents a solid polymer support, suitably derivatized to link to a ribonucleotide unit;

(RNu) represents a ribonucleotide unit linked to the polymer support by means of its 5'-position,

(DNu) represents a 3', 5'-linked deoxyribonucleotide chain of predetermined unit sequence and chain length, m units in length, and linked to (RNu) through a 2'-3' or a 3'-3' bond;

and m is an integer of at least 3;

The initial ribose unit, to assume the -position in the growing chain, is linked directly to the polymer chain through its 5'-hydroxyl, so that either of its 2' or 3'-hydroxyl group is available for linkage to the first deoxyribose unit. It is necessary to utilize the 5'-position of the ribose unit for polymer bonding, not only to ensure high initial polymer loadings and consequent high end product yields, but also to provide ready cleavage of the ribose unit from the remaining, deoxyribo unit chain. The linkage of the $\alpha$-position ribose unit to the first deoxyribose unit is 2'-3' or 3'-3', so that, after suitable deprotection, the ribose unit has either a 2'-hydroxyl or a 3'-hydroxyl in close proximity to the phosphate group linking the ribose deoxyribose units, to provide for ready cleavage therebetween.

The resulting product is a 5'-DNA-3' chain sequence, produced simply and efficiently by stepwise chain extension, in high yield. The product may be separated and purified by standard, known techniques, normally using chromatographic methods.

It will be appreciated that similar results cannot be obtained using initial deoxyribose units linked to the polymer chain. Whilst it might be possible to get high polymer-nucleoside initial loadings by using the 5'-position of a deoxyribo unit to link to the polymer, all subsequent reactions would entail use of the less reactive 3'-position of the polymer linked product, with consequent inconveniences, longer reaction times and reduced yields.

Suitable solid polymer supports for use in the process of the invention are insoluble in the chosen reaction medium, have a degree of rigidity, surface impermeability, and a stability sufficient to withstand subsequent reaction conditions. They are used in the form of solid powders or granules. A very wide choice of different polymers is available for this use. They should have reactive surface side groups which can be chemically modified to derivatize the polymer and put in a side group having at least six atoms linearly arranged, and having a terminal carboxylic acid or carbonyl halide group. Thus, hydroxyl containing polymers are suitable and preferred, since the hydroxyl can be reacted to form a chain with terminal acidic (acid or acid halide) groups by suitable, relatively straightforward chemical processes. The polymers may be organic polymers having polyolefin chains such as polystyrene, polyacrylates, polymethacrylates and polyvinyl alcohol. They may be polydiolefins, or condensation polymers such as polyamides, polyesters, polyurethanes, or polyamide-imides. They may be other

types of polymers such as polypeptides, cellulose, polysaccharides and the like. They may be organic polymers such as silica gel. They must have suitable chemical groups to allow attachments to a nucleoside and removal therefrom.

The linkage formed between the polymer and the initial nucleoside unit is an ester-type linkage, using a predetermined hydroxyl group of the nucleoside and the acid group present on the side chain of the derivatized polymer. Thus in the case of a polyhydroxy polymer such as cellulose, polyvinyl alcohol or silica gel, a suitable polymer derivatization process might involve, firstly, treatment with $\gamma$-aminopropyltriethoxy-silane or other suitable reagent to form amine terminated side groups on the polymer, followed by reaction with a difunctional acid compound, e.g. succinic anhydride, to form acid group terminated polymer side chains with intermediate amide chain linkages.

Other methods of reactions of hydroxyl groups on the polymer to put on acid functions are known in the art, and are useful in the process of the present invention. One such method in the reaction of the hydroxyl groups with a half-ester of a dicarboxylic acid, for example, adipic acid half ester, pimelic acid half ester, suberic acid half ester, azelaic acid half ester and sebacic acid half ester. Then, after hydrolysis to convert the residual ester groups back to acid groups, a derivatized polymer with acid terminated side groups of suitable chain length, and containing an ester linkage is formed ready for use in the present invention.

A further alternative reaction is to treat the hydroxyl bearing polymer with a shorter chain diacid or diacid anhydride, e.g. succinic anhydride, followed by reaction with a polyalcohol of suitable chain length, thereby forming a polymer derivative

having side chains of suitable length for use in the present invention, with intermediate ester linkages, and terminal hydroxyl groups. Subsequent treatment with succinic anhydride, for example, puts on the required acid terminal group.

In another alternative method, a hydroxyl-bearing polymer may be treated with a halobenzyl acid or ester compound, or a halo-trityl acid or ester compound to produce a polymer having an ether linkage in the side chain, optionally hydrolysable to put terminal acid groups thereon.

It is not necessary to start with hydroxyl-bearing polymers in the process of the present invention, although these are preferred. It is, however, most preferred that the polymer be capable of derivatization to put on a group -X-COOH or -CO-Hal, in which X represents a chain having at least six linearly arranged atoms therein and comprising carbon-carbon chains optionally interrupted by one or more linkages selected from ester linkages, amide linkages and ether linkages. Polystyrene is another preferred polymer, since it can be obtained with a suitable degree of cross-linking to obtain the required surface impermeability. Methods of derivatizing polystyrene and similar aromatic side group bearing polymers, to put acid or halocarbonyl groups thereon are known. One such method involves a Friedel-Craft reaction using aluminum trichloride and succinic anhydride, resulting in a polymer having the group -X-COOH attached to a side aromatic group.

In one preferred embodiment of the present invention, the polymer-linking group X in formula (I) is a ribonucleoside unit, linked to the polymer via an ester linkage to the 5'-position on the ribonucleoside. Then the product intermediate has ribo unit at or adjacent to each chain end.

In order to ensure that the ribose joins to the polymer through its 5'-position, the ribose at the α-position is appropriately 2' and/or 3'-protected prior to reaction with the polymer. Suitable protecting groups are well-known and include silyl groups, trityl groups, alkyl groups and the like. It is normally necessary only to protect one of the 2' and 3' positions, since the introduction of a relatively bulky protecting group at such a position render the other positioned hydroxyl unreactive, by steric hindrance. A preferred protecting process is reaction of the ribose compound with methyl orthoacetate, which protects both the 2' and the 3' positions.

In the next stage of the process, the ribonucleoside attached to the polymer is deprotected at either or both its 2' and 3' position, e.g. by reaction with acid, and the product is ready for chain extension with deoxyribonucleotide units, the link to the first of such units being 2'-3' or 3'-3'. In a later step, the initial ribonucleoside unit is removed, and so the precise linkage position is immaterial. Either of these linkages is readily susceptible to chemical or enzymatic reaction to cleave the ribonucleotide unit from the chain.

Chain extension with deoxyribo units can now take place, in the manner described in aforementioned U.S. patent application 149,865 Ogilvie et al, to form 3'-5' linked DNA chains of predetermined unit sequence and length, or by other known methods. Preferably the units which are reacted are deoxribonucleotide units, with methyl phosphodichloridite jointed at their 3'-position, and their 5'-position protected e.g. with suitable chemically inert removable protecting groups such as monomethoxytrityl. The protecting group is removed from the 5'-position after the unit has been linked to the polymer supported polynucleotide chain.

At a convenient, subsequent stage, the polynucleotide is oxidized, e.g. with iodine in water, to oxidize the phosphite linkages to phosphate groups. After such chain extension step, it may be desirable to treat the product with a hydroxyl capping reagent, to prevent further chain growth of undesired, unreacted products from each intermediate step.

Once the desired DNA sequence has thus been prepared, to the desired chain length, at least three terminal ribonucleotide units are attached to the end of the chain, according to one preferred embodiment of the invention. These may be the same as or different from the ribonucleotide unit used in the α-chain position. Preferably all of the at least three terminal ribonucleotide units are identical. The sequence is suitably added to the chain in a stepwise, chain extension manner, as in the case of deoxyribose chain extensions, using suitably protected reagents and reaction steps, to ensure proper 3'-5' linear chain extension.It is possible to preform the desired ribonucleotide sequence and couple it to the polymer-supported chains, but this method is inefficient and consequently less preferred.

Products of formula I given above are themselves useful as affinity columns, for the separation of mixtures of nucleotide and polynucleotide products. Similarly, products of formula II can be used to link to solid polymer such as cellulose and cellulose derivatives to form affinity columns for separating nucleotide and polynucleotide products.

It is noteworthy that polymer supported polynucleotide chains which have not chain extended to the full, predetermined sequence in the previous steps will not react to add on the terminal ribonucleotide sequence. Such unextended chains are to all intents and purposes inactive and unextendible further. In

any event, as a safeguard against further "restarting" of these chains at a subsequent stage, to give improper sequences, they may be capped by treatment with hydroxyl deactivating groups (acids, acid halides, isocyanates, etc.) as noted above. As a result, the only products in the mixture which can add the terminal ribo sequence are those of properly extended, predetermined unit sequence and length.

Next, the product of general formula I given above, must be cleaved from the polymer and separated from other reaction products, e.g. improperly extended chains and the like. Treatment with weak alkali is sufficient to cleave the ester bond and separate the product from the polymer support. A specific preferred such base for this purpose is t. butylamine, which in addition to cleaving the product from the polymer, at the same time serves to remove the methyl protecting groups from the phosphite or phosphate links. A product of general formula II is thus formed. It is separated from other reaction byproducts by use of an affinity column.

Appropriate affinity columns are commercially available, and comprise oligonucleotides attached to solid polymer (normally cellulose or cellulose derivative polymers). The oligonucleotide on the polymer is chosen appropriately to pair with the ribonucleotide ($RNu_1$) forming the terminal sequence on the product. These pairings are the well-known pairings of respective bases from nucleic acid chemistry, namely, adenine-thymine; adenine-uracil; and cytosine-guanine. It is not substantially material whether the affinity column has ribose units or deoxyribose units thereon. Since commercially available affinity columns in general have homopolymeric sequences of ribose or deoxyribose units, it is preferred that all units (RNu) in the products of the invention be the same.

The method of operation of the separation using the affinity column is standard, and does not require detailed description. Basically, the product mixture in an aqueous buffered solution is poured down the column, and all components excepting that having the pre-arranged terminal ribo sequence pass through. The desired, retained product is subsequently recovered from the columns by solvent elution, suitably using aqueous buffered phosphate solution.

Finally to recover the desired product, the ribo units must be removed. This can readily be accomplished either enzymatically or chemically. Ribonuclease enzymes are well known, and readily available, for effecting cleavage of ribonucleotide units from deoxyribonucleotide chains. Alternatively, treatment with strong alkali will effect chain cleavage to remove the ribo units and produce the DNA oligonucleotide product of predetermined sequence and chain length. The product is recovered and purified in the normal way.

Whilst the preferred process of the present invention adds the sequence of units $(RNu)_n$ as the final series of chain extension steps to the polymer supported chain, it is possible to adopt the reverse procedure and add the sequence of ribose units initially, to the polymer support. The first deoxyribonucleotide unit is then added to an intermediate product of the form:

$$(Pol)-X-(RNu)_n$$

via a 3'-3' or a 2'-3 link. After all chain extension steps and cleavage from the polymer support, a product of general formula $(DNu)_m-(RNu)_n-Y$, closely resembling general formula (II) is again obtained.

The invention is further described for illustrative purposes in the following specific examples.

EXAMPLE 1

A silica gel polymer support is prepared and derivatized, then linked firstly to a ribonucleotide unit and subsequently a DNA chain synthesized stepwise thereon in a column. The polymer support used is Vydak TP silica, a particularly suitable rigid non-swellable polymer. The polymer is first activated by refluxing HCl and then derivatized successively with 3-aminopropyltriethoxysilane, succinic anhydride and trimethylchlorosilane, as previously described.

In the meantime, 2', 3'-O-methoxymethyl-ideneuridine, of formula

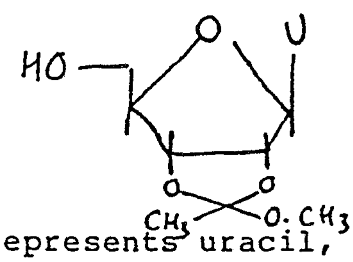

wherein U represents uracil, was prepared by the method of Reese, "Tetrahedron Letters", 23 2301,(1967), involving essentially reaction of uridine with methylorthoacetate.

To couple the 2',3'-protected uridine to the derivatized polymer, 10g of polymer and 0.75 g of uridine compound are just covered with pyridine, and the pyridine was evaporated on the high vacuum pump. More pyridine (30ml) is added, and 5ml thereof evaporated on the high vacuum pump. Then DCC(2.5g) is added followed by a small amount of 4-dimethylaminopyridine. The flask containing the reactants is then stoppered and shaken at room temperature for 20 hours. After being allowed to stand for a brief period, the pyridine solution is decanted by aspiration. Pyridine (15ml) is added, followed by p-nitrophenol (0.425 g) and DCC (1 g) to cap off unreacted polymer acid groups, and the flask shaken at room temperature for about 4 hr. Then 20 ml piperidine is added and the flask shaken for a further approximate 20 hour period. Then the material is

filtered and washed successively with pyridine, ethanol, acetone and ether, three times with each liquid. A small portion of the product is analysed to determine extent of reaction, and it is found that the polymer support has approximately 90  moles per gram of linked uridine product.

The polymer-supported ribonucleoside thus prepared is then used to prepare an oligodeoxyribonucleotide sequence, twelve units in length, of sequence

5' GAGATAATGTTT.3'

The three nucleotide units for stepwise sequential addition are preformed, by amino-protecting the purine-pyrimidine base units, 5'-tritylating the deoxyribose nucleus for protection purposes (monomethoxy trityl), and then reacting the product with methylphosphodichloridite, $CH_3$-O-P $(Cl)_2$ to form a 3'-phsphorylated derivative, of general formula II

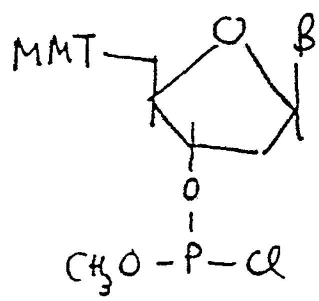

where B' represents the protected base group. This preparation is performed in the standard, known way.

The polymer supported product is introduced into a reaction column and deprotected by reaction with acid, to restore OH groups at one of position 2' and 3'. washed, and the first phosphorylated nucleoside II, namely the thymine derivative is introduced into the column for condensation. After this the product on the polymer is washed, treated with phenylisocyanate to remove any water adhering to the polyer and to cap any unreacted hydroxyl groups, deprotected at the 5' position with tetrafluoracetic

acid, washed again and the next nucleotide unit II introduced in this case another thymine derivative, and coupled into the chain. By repeat of these steps the above nucleotide sequence is built, from the 3' end, by 3'-5' coupling to the ribonucleoside-polymer support.

Finally the product is treated in the column with t.butylamine, which separates the product from the polymer support. Then the product is treated with ammonium hydroxide which removes the α ribo-unit. The product is recovered by filtration, washed and purified by standard techniques. On analysis, it proves to be the oligonucleotide of sequence given above, and terminated by 5'-hydroxyl on the guanosine end and 3'-hydroxyl on the thymine end.

EXAMPLE 2

The methods, materials and procedures of Example 1 are repeated, until the 12-unit sequence, coupled to the polymer through a ribose unit at the α-position, has been prepared. Then, instead of separating it from the polymer at this stage, it is reacted in its next step with 2',3'-o-methoxymethylidene-uridine, in the presence of methyl phosphodichloridite, by the procedure described previously, and then the product is deprotected with acid, and chain extended with eight adenosine units by the phosphite procedure, with appropriate protection to ensure 3'-5' linkage. The reactions are conducted stepwise to ensure a linear chain extension with eight linearly arranged units. The procedure is conducted in the column, as generally previously described.

Then the product is treated in the column with t.butylamine, which cleaves the ester linkage to separate the product from the polymer support, and removes the methyl blocking groups from the phosphate linkages. The product is further reacted with

trifluoroacetic acid, to remove the 5'-trityl protecting group from the terminal adenosine unit.

Next, the product mixture is separated, using an affinity column of cellulose, having attached thereto sequences of ten thymidine units. Standard procedures are adopted, mixing the product mixture with buffered phosphate solution, passing the mixture down the column and collecting and discarding liquid products using from the bottom of the affinity column.Then, according to standard procedures, the affinity column is washed with an aqueous buffered phosphate solution containing 0.15 M lithium chloride to remove the solid product from the column. The product as recovered is treated in aqueous solution under appropriate, standard conditions with the enzyme ribonuclease, which effects chain cleavage to remove the ribo units from each end of the chain.

There is recovered the dodecamer of deoxyribonucleotide, which is washed and dried, and on testing proves to be pure and consisting of the predetermined unit sequences only.

WE CLAIMS.

1. An oligonucleotide of predetermined unit sequence and chain length and comprising predominantly 3'-5' linked deoxyribonucleotide sequences but having at least one ribonucleotide unit chemically linked thereto at or adjacent to one end of the nucleotide chain, said oligonucleotide corresponding to the general formula:

$$(DNu)_n - RNu - (Nu)_m$$

wherein: $(DNu)_n$ represents a 3'-5' linked deoxyribonucleotide sequence of predetermined unit sequence and length, optionally chemically protected, n deoxyribonucleotide units in length;

n is an integer from 3 to about 200;

RNu represents a ribonucleotide units;

Nu represents a sequence of either deoxyribonucleotide units or ribonucleotide units, of predetermined unit sequence and chain length, optionally chemically protected, m units in length;

and m is an integer from 0 to 8.

2. A process of preparing oligoribonucleotides of pre-determined unit length and sequence, which comprises preparing, by stepwise chain extension reaction, a reaction mixture containing a compound of general formula:

$$(DNu)_m\text{-RNu-}(pNu)$$

wherein (DNu) represents a predetermined sequence of deoxyribonucleotide units linked together by means of 3'-5' phosphate linkages, and m units in length,

m is an integer of at least 3;

RNu represents a ribonucleoside or ribonucleotide unit;

(pNu) represents either a predetermined sequence of at least two 3'-5' linked ribonucleoside or ribonucleotide units, or a predetermined sequence of at least three 3'-5' linked deoxyribonucleoside or deoxyribonucleotide units;

passing the reaction mixture containing said compound down an affinity column which has attached thereto nucleotide or nucleoside unit sequences, at least three units in length, showing selective affinity for the (pNu) sequence of the compound, so as to retain the compound selectively on the column in separation from the other reaction mixture components;

recovering the compound separately from the affinity column,

treating the compound so recovered to cleave the RNu-(pNu) sequence from the (DNu) sequence,

and recovering the oligoribonucleotide $(DNu)_m$.

3. The process of claim 2 wherein the compound $(DNu)_m$ -RNu-(pNu) is prepared by a polymer support process, in which nucleotide units are added sequentially to a solid polymer-supported nucleotide chain for stepwise chain extension in predetermined order.

4. The process of claim 3 wherein the initial nucleotide unit is linked to the polymer support through an ester linkage.

5. The process of claim 3 or claim 4 wherein the initial nucleotide unit is a ribose unit, linked to the polymer support through its 5' position and linked to the succeeding deoxyribose unit through a 2'-3' or a 3'-3' linkage.

6. The process of claim 3, claim 4 or claim 5, wherein the derivatized solid polymer has the general formula

(Pol)-Z-COM

in which Z represents a chain having at least six linearly arranged atoms therein and comprising

carbon-carbon chains optionally interrupted by one or more linkages selected from ester linkages, amide linkages and ether linkages, M is OH or halogen, and (Pol) represents a solid organic polymer or polymer of silica.

7. The process of any of claims 2 or 6 wherein the RNu-(pNu) sequence is cleaved from the $(DNu)_m$ sequence by treatment with ribonuclease.

8. The process of any of claims 3-6 wherein the oligonucleotide is cleaved from the polymer support by treatment with t.butylamine.

9. An oligonucleotide compound of general formula

$(DNu)_m$ - RNu-(pNu)

wherein $(DNu)_m$ represents a predetermined sequence of 3'-5' linked deoxyribonucleotide units, m units in length,

m is an integer of at least 3;

RNu represents a ribonucleotide or ribonucleoside unit;

and (pNu) represents either a predetermined sequence of at least two 3'-5' linked ribonucleoside or ribonucleotide units, or a predetermined sequence of at least three 3'-5' linked deoxyribonucleoside or deoxyribonucleotide units.

10. The compound of claim 9 wherein m is an integer of from about 6 to about 200.

11. A polymer supported oligonucleotide compound of general formula

(Pol) - X - $(DNu)_m$-RNu-(pNu)

wherein (Pol) represents a solid derivatized polymer, X represents a linking group chemically linking a deoxyribo-nucleotide unit to the polymer and including an ester linkage, and (DNu), RNu, (pNu) and m have the meanings given in claim 9.

12. The compound of claim 11 wherein X comprises a ribo-nucleotide unit joined to the polymer from its 5' position via said ester linkage.

13. A process of preparing oligodeoxy-ribo-nucleotides, which comprising coupling to a solid polymer support a ribonucleoside unit via the 5'-position of the ribonucleoside, chain extending the polymer supported ribonucleoside, from its 2' or its 3'-position in stepwise fashion with a sequence of deoxyribonucleotide units, cleaving the solid polymer support and removing the ribonucleoside unit from the chain.

14. The process of claim 13 wherein the ribonucleoside unit is coupled to the polymer support through an ester linkage to the 5'-position of the ribonucleoside, and the first deoxyribonucleotide unit is joined to the ribonucleoside unit through its phosphate or phosphite linkage joining the 3'-position of the deoxyribonucleotide to the 2' or 3'- position of the ribonucleoside unit.

15. The process of claim 14 wherein subsequent deoxyribonucleotides are linked to one another to form 5',3'-linear nucleotide chains.

16. The process of any of claims 13-15 wherein the polymer support is derivatized silica gel.

17. The process of any of claims 13-16 wherein cleavage from the polymer support is achieved by treatment with t.butylamine.

18. The process of any of claims 13-17 wherein the ribonucleotide unit is subsequently removed from the chain by treatment with alkali.

19. The process of any of claims 13-17 wherein the ribonucleotide unit is subsequently removed from the chain by treatment with RNAse.

20. The process of any of claims 13-16 wherein the product is cleaved from the polymer support and the ribonucleotide is removed from the chain in a single stage by treatment with strong base.

21. An oligonucleotide-solid polymer linked complex of general formula:

$$Pol - (RNu) - (DNu)_m$$

wherein Pol represents a solid polymer support suitably derivatized to link to a ribonucleotide unit linked to the polymer support by means of its 5'-position;

$(DNu)_m$ represents a 3', 5'-linked deoxyribonucleotide chain of predetermined unit sequence and chain length, m units in length, and linked to (RNu) through the 3'-position on the first (DNu) unit;

and m is an integer of at least 3.

0067597

European Patent Office

# EUROPEAN SEARCH REPORT

Application number

EP 82 30 2775

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | STRUCT. ORDER POLYM. LECT. INT. SYMP. 1980, Publ.1981, C. Francesco Pergamon, Oxford (GB) M.H. CARUTHERS: "Synthesis of sequence defined polynucleotides via diester, triester and phosphite coupling methods", page 111 * page 118, last three sentences * & Int. Symposium on macromolecules sept.1980<br>--- | 1,2 | C 07 H 21/00 |
| Y | TETRAHEDRON, vol.34, no.56, 1978, Pergamon Press Ltd., (GB) C.B. REESE: "The chemical synthesis of oligo- and poly-nucleotides by the phosphotriester approach", * page 3144, left-hand column, second paragraph *<br>----- | 1,2 | |
| | | | TECHNICAL FIELDS SEARCHED (Int. Cl. 3) |
| | | | C 07 H 21/00 |

The present search report has been drawn up for all claims

| Place of search | Date of completion of the search | Examiner |
|---|---|---|
| THE HAGUE | 14-09-1982 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document